(19) Europäisches Patentamt
European Patent Office
Office européen des brevets

(11) Publication number: **0 383 531 B1**

(12) **EUROPEAN PATENT SPECIFICATION**

(45) Date of publication of patent specification: **10.08.94**

(51) Int. Cl.5: **C07D 473/00**

(21) Application number: **90301490.0**

(22) Date of filing: **13.02.90**

# (54) 6'-deoxy-6'-halogenoneplanosin A and its production.

(30) Priority: **14.02.89 JP 34748/89**

(43) Date of publication of application:
**22.08.90 Bulletin 90/34**

(45) Publication of the grant of the patent:
**10.08.94 Bulletin 94/32**

(84) Designated Contracting States:
**DE FR GB IT**

(56) References cited:
**EP-A- 0 304 889**
**FR-A- 2 500 838**

(73) Proprietor: **ASAHI KASEI KOGYO KABUSHIKI KAISHA**
**2-6, Dojimahama 1-chome**
**Kita-ku**
**Osaka (JP)**

(72) Inventor: **Shuto, Satoshi**
**250-8 Naka**
**Mishima-shi, Shizuoka (JP)**
Inventor: **Obara, Takumi**
**632-1, Mifuku,**
**Ohito-cho**
**Tagata-gun, Shizuoka (JP)**
Inventor: **Itoh, Hiromichi**
**38-2 Sankei-dai**
**Mishima-shi, Shizuoka (JP)**
Inventor: **Koshio, Takehiro**
**632-1 Mifuku,**
**Ohito-cho**
**Tagata-gun, Shizuoka (JP)**
Inventor: **Fujiwara, Tatsuro**
**310 Nirayama,**
**Nirayama-cho**
**Tagata-gun, Shizuoka (JP)**
Inventor: **Yaso, Masao**
**848-1 Takyo,**
**Ohito-cho**
**Tagata-gun, Shizuoka (JP)**

(74) Representative: **Woods, Geoffrey Corlett et al**
**J.A. KEMP & CO.**
**14 South Square**
**Gray's Inn**
**London WC1R 5LX (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

This invention relates to neplanosin A derivatives having antitumor activity and to salts thereof.

Neplanosin A is a compound of formula:

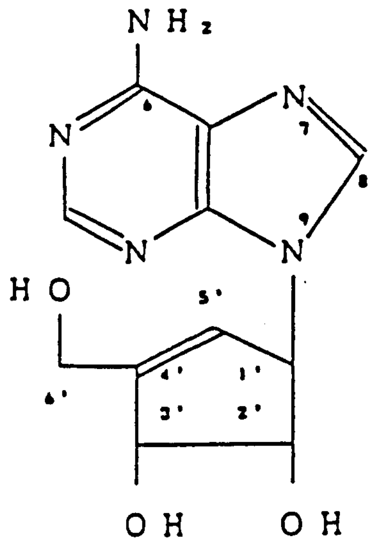

which has cytotoxic activity in vitro and growth inhibiting activity against transplanted mice ascites tumors in vivo [Proceeding of 11th International Congress of Chemotherapy, Vol.2, 1559-1561 (1979)] (hereinafter designated as ref.1). However its antitumor activity is not sufficient for use as an antitumor agent. Derivatives of neplanosin A have also been synthesized (JP-A-57-163383, JP-A-57-102889, JP-A-58-85898, JP-A-58-118586, JP-A-58-183691 and JP-A-59-219284). However no derivatives having superior properties over neplanosin A have been found.

The cytotoxic activity (antitumor activity) of neplanosin A has been revealed, after phosphorylation of the hydroxyl group at position 6 thereof, to be an action of kinase in the cells [Cancer Res.,46: 1063-1067 (1986)] (hereinafter designated as ref.2). It is therefore thought that there is no action on tumor cells having no kinase activity for neplanosin A when used as a substrate. Furthermore, the tumor activity of neplanosin A in vivo is thought to be reduced by the action of adenosine deaminase, which is widely distributed in vivo, which rapidly converts the neplanosin A to an inert deaminated compound (neplanosin D) (ref.1).

Therefore derivatives of neplanosin A which are not inactivated by the action of adenosine deaminase are strongly desired.

The present invention provides a compound of formula (1):

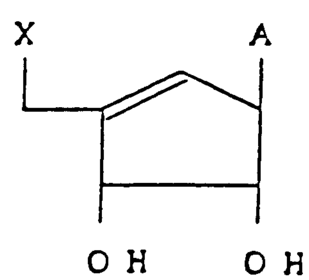

wherein A is an adenine-9-yl group and X is a halogen, or a pharmacologically acceptable non-toxic salt thereof.

The 6′-deoxy-6′-halogenoneplanosin A of formula (1) is not inactivated by adenosine deaminase and has an almost equal level of cytotoxic activity to that of neplanosin A. Moreover the compounds of the present invention have no hydroxyl group at position 6 which can be affected by enzymatic phosphorylation. However they quite surprisingly have an equal level of cytotoxicity to neplanosin A, and are therefore useful antitumor agents.

Examples of the salt are inorganic salts such as hydrochloride, sulphate or phosphate and organic salts such as acetate, propionate, tartrate, citrate, glycolate, gluconate, succinate, malate, glutamate, aspartate or methanesulfonate. Other non-toxic salts with organic acids are also, for example, possible.

The present invention also provides a process for the production of a compound as defined above which comprises substituting the hydroxyl group at position 6' in neplanosin A or 2' and/or 3'-O-protected neplanosin A with a halogen by a halogenating agent, and, when the hydroxyl groups at positions 2' and/or 3' are protected, removing the protecting group thereof, and, if desired, converting the thus obtained compound of formula (I) to a pharmacologically acceptable non-toxic salt thereof.

The present invention also provides a process for the production of a compound as defined above wherein X is iodine (a compound of formula 1c) which comprises substituting the halogen at position 6' in a compound of formula:

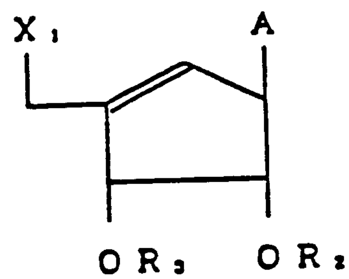

wherein A is adenine-9-yl, X is chlorine or bromine and $R_2$ and $R_3$, which may be identical or different, are hydrogen or a hydroxyl protecting group, with iodine by an iodinating agent, and, when the hydroxyl groups at position 2' and/or 3' are protected, removing the protecting group thereof, and, if desired, converting the thus obtained compound of formula (I) in which X is iodine to a pharmacologically acceptable non-toxic salt thereof.

The above 2' and/or 3'-O-protected neplanosin A is a compound wherein the hydroxyl group at position 2' and/or 3' in neplanosin A is protected by a known protecting group used in nucleic acid chemistry. An example of the protecting group is a ketone compound residue in which a cyclic acetal is formed with two oxygen atoms adjacent to each other, for example isopropylidene, methoxymethylene, methoxyethylidene, ethoxymethylene, benzylidene and cycloalkylidene. These protecting groups can be introduced by a reaction with a corresponding aldehyde or ketone compound in the presence of an acid catalyst. The hydroxyl at position 2' and/or 3' can also be protected by an acyl group such as formyl, acetyl, chloroacetyl, trichloroacetyl, trifluoroacetyl, methoxyacetyl, pivaloyl, benzoyl, $\beta$-benzoylpropionyl or trityloxyacetyl, a trityl group such as trityl, monomethoxytrityl, dimethoxytrityl or trimethoxytrityl, or methoxymethyl.

In the present invention, the compound of formula (I) is preferably produced by the following process according to the nature of X.

(A) A process for production of a compound of formula (1a) wherein X is Cl or Br:

A compound of formula (1a) can be produced by halogenating neplanosin A with a halogenating agent using triphenylphosphine and $CCl_4$ or $CBr_4$ in a solvent. Examples of solvents are organic solvents such as dimethylformamide and hexamethylphosphoric triamide. The halogenation reactions proceeds, in general, at room temperature. The reaction process can be traced by silica-gel thin layer chromatography (TLC) or high performance liquid chromatography (HPLC), so that the reaction can be terminated when maximum production of the compound of formula (1a) is observed.

Isolation of the compound of formula (1a) can be performed by stopping the reaction by adding an alcohol such as methanol, removing the solvent, adding a solvent such as chloroform, and filtering the thus formed insoluble product of formula (1a).

Another method of halogenation is to use a halogenating agent such as $SOCl_2$ or $SOBr_2$. A compound of formula (1a) wherein X is Cl is preferably produced using $SOCl_2$. The thus produced compound of formula (1a) can be purified by column chromatography using silica-gel or an adsorption resin with an elution solvent such as chloroform-methanol.

(B) A process for production of a compound of formula (1b) wherein X is F:

A compound of formula (1b) can be produced by fluorinating 2', 3'-O-protected neplanosin A (2) with a fluorinating agent using diethylamino sulfatrifluoride in a reaction solvent, and removing the hydroxyl protecting groups at positions 2' and 3'.

Examples of the above reaction solvent are organic solvents such as dichloromethane, chloroform or dichloroethane. The fluorinating reaction can proceed under ice-cooling. The reaction process can be traced by TLC or HPLC, so that the reaction can be terminated when the maximum production of the compound of formula (1b) is observed.

Isolation of the thus obtained 6'-deoxy-6'-fluoro-2',3'-O-protected neplanosin A (3) can be performed by adding an aqueous alkali such as sodium bicarbonate solution to stop the reaction, adding an extraction solvent such as chloroform, filtering the insoluble material, separating the filtrate, and concentrating the organic layer. Further purification can be made by column chromatography using silica-gel or an adsorption resin with an elution solvent such as chloroformmethanol.

A compound of formula (1b) can be obtained by removing the hydroxyl protecting groups at positions 2' and 3' in the product of formula (3) by a known removal used in nucleic acid chemistry. For example, isopropylidene can be removed by treatment with an aqueous acid such as aqueous formic acid or acetic acid.

Isolation of the thus obtained compound of formula (1b) can be performed by removing the aqueous acid, treating the residue with an organic solvent such as tetrahydrofuran for crystallization, or by column chromatography using silica-gel or an adsorption resin with an elution solvent such as chloroform-methanol.

[C] Process for production of a compound of formula [1c] wherein X is I:

A compound of formula [1c] can be produced by iodinating a halogen at position 6' in the compound of formula [1a] with an iodinating agent using an alkaline metal iodide in a reaction solvent.

Examples of reaction solvents are organic solvents such as acetonitrile. Examples of the alkaline metal iodide are LiI or NaI. The iodination reaction can in general be performed while refluxing the reaction solvent.

Isolation and purification of the compound of formula [1c] can be made by removing the reaction solvent, dissolving the residue in methanol, and subjecting the compound to column chromatography using silica-gel or an adsorption resin elution solvent such as chloroformmethanol.

A salt of the thus obtained compound of formula [1] can be prepared, if desired, by a known method.

The present invention also provide a compound of formula [1], or a pharmacologically acceptable non-toxic salt thereof, for use in a method of treatment of the human or animal body by therapy, in particular for use as an anti-tumour agent.

The present invention additionally provides the use of a compound of formula [1], or a pharmacologically acceptable non-toxic salt thereof, in the manufacture of a medicament for use as an anti-tumour agent.

The present invention further provides a pharmaceutical composition which comprises a compound of formula [I], or a pharmacologically acceptable salt thereof, and a pharmaceutically acceptable diluent.

Growth inhibition activity against L5178Y cells and resistance to adenosine deaminase of a compound of formula [1] of the present invention are now illustrated:

(1) Growth inhibition activity on L5178Y cells: (Method)

A solution of test compound is prepared by dissolving or suspending the compound in RRMI 1640 medium containing 10% bovine serum by ultrasonication. A cell suspension of L5178Y cells, 1.1 x $10^4$ cells/ml, each 1.8 ml, is separately added in a test tube. The solution (0.2 ml) of the test compound hereinabove is added therein and the medium is tightly sealed and incubated at 37°C for 48 hours. After incubation, the number of cells is measured using a Coulter counter and the growth ratio of cells are determined.

The results are shown in Table 1:

Table 1

| Growth inhibition against L5178Y cell | | |
|---|---|---|
| Test sample | | $IC_{50}$ (μg/ml) |
| Control | neplanosin A | 0.2 |
| Compound (1) | X = Cl | 0.3 |
| | X = Br | 0.2 |
| | X = F | 1.1 |
| | X = I | 0.4 |

(2) Resistance to adenosine deaminase:

[Test method]

A solution (0.5 mM) of a test sample is prepared by dissolving the test sample in 0.05 M Tris-HCl buffer (pH 7.2). Adenosine deaminase (Calf intestine, 400 U/ml glycerin, Boehringer 102091) is diluted ten times with 0.05 M Tris-HCl buffer (pH 7.2) to prepare an adenosine deaminase solution.

The above sample solution (0.5 ml) is mixed with adenosine deaminase solution (10 μl). The mixture is allowed to stand in a water bath at 25°C, and samples (each 4μl) thereof are pipetted off after 0, 10, 20 and 30 minutes. The remaining sample is measured by HPLC according to the following conditions:

HPLC:

Column: Licrosorb RP-18-5
Elution solvent: Control: 15% methanol
Compound; 25% methanol
Elution speed: 1.0 ml/min.
Temperature: 50°C
Detection: 260 nm UV
Injection volume: 4 μl

Retention time:

| | | |
|---|---|---|
| Control: | | 2.2 min |
| Compound (1); | X = Cl:<br>X = Br:<br>X = F:<br>X = I: | 3.9 min<br>4.5 min<br>2.4 min<br>6.5 min |

The results are shown in Table 2:

Table 2

| Test Sample | | Remaining amount (%) | | |
|---|---|---|---|---|
| | | 10 min | 20 min | 30 min |
| Control Neplanosin A | | 6 | 0.4 | 0 |
| Compound (1) | X = Cl | 101 | 99 | 102 |
| | X = Br | 98 | 97 | 97 |
| | X = F | 99 | 98 | 98 |
| | X = I | 97 | 96 | 96 |

As shown in the above, a compound of formula (1) of the present invention has growth inhibitory activity against L5178Y cells at almost the same level as neplanosin A. However the compound of formula (1) is not decomposed by adenosine deaminase even after 30 minutes of incubation and hence it has very high resistant activity which can not be compared with neplanosin A. The cytotoxic action of neplanosin A is said to proceed via enzymatic phosphorylation of hydroxyl at position 6'. The compound of formula (I), having no hydroxyl group at position 6', which is to be phosphorilated, unexpectedly has cytotoxic activity. Hence it is useful as an antitumor agent.

## Examples

The following Examples further illustrate the present invention.

## EXAMPLE 1

Production of 6'-deoxy-6'chloroneplanosin A:

Hexamethyl phosphoric triamide (HMPA) (1 ml) and thionyl chloride (150 µl) were added to neplanosin A 80 mg (0.3 m mol) and the mixture was stirred at room temperature for 2 hours. The reaction mixture was charged on a column (2 x 5 cm) of silica-gel (Wako Pure Chem. Co., C-200) and eluted with chloroform, chloroform-methanol (20:1) and chloroform-methanol (5:1) in this order. A fraction eluted with chloroform-methanol (5:1) was concentrated in vacuo. Isopropanol was added to the residue, concentrated twice in vacuo and the residue was kept in a refrigerator overnight. The product, dissolved in a small amount of methanol, was neutralized with 0.5 N sodium bicarbonate solution and the solvent was removed. The residue was purified by preparative chromatography using a silica-gel plate (Merck, Art 5717) [developer: chloroform-methanol (6:1), elution: chloroform-methanol (2:1)) and recrystallized from ethanol to obtain the compound (52 mg).

| Elemental analysis [$C_{11}H_{12}N_5O_2Cl \cdot 1/2\ H_2O$ ] : | | | | |
|---|---|---|---|---|
| Theoretical:<br>Found: | C: 45.45,<br>C: 45.03, | H: 4.85,<br>H: 4.54, | N: 23.37,<br>N: 23.31, | Cl: 11.83<br>Cl: 11.87 |

UV ; λmax 260 nm (water)

$^1$H-NMR (400 MH$_z$, $CD_3$ OD) δ ; 4.34 (t, 2H, H-6' )、 4. 43 (dd, 1H, H-2' , $J_{2',1'}$ = $J_{2',3'}$ = 5. 37 Hz)、 4. 73 (d, 1H, H-3' , $J_{2',3'}$ = 5. 37 Hz)、 5. 50 (m, 1H, H-1')、 6. 08 ( dd, 1H, H-5' , J = 1. 5, 3. 5 Hz)、 8. 08 (s、 1H, H-2)、 8. 19 (s, 1H, H-8)

TLC ; Rf = 0. 28 [Merck, Art 5715 plate developing solvent; Chloroformmethanol (5 : 1)

## Example 2

Production of 6'-deoxy-6'-chloroneplanosin A:

Triphenylphosphine 341 mg (1.3 m mol) and carbontetrachloride 145 µl (1.5 m mol) were added to neplanosin A 263 mg (1 m mol) suspended in dimethylformamide (6 ml) and stirred at room temperature for 30 minutes. The same amounts of triphenylphosphine and carbon tetrachloride as above were added thereto and stirred for 2 hours. Methanol (5 ml) was added to the reaction mixture, stirred for 30 minutes and the solvent was distilled off in vacuo. Chloroform was added to the residue and the insolubles were collected by filtration. The insoluble material was dissolved in a small amount of methanol. The solution, mixed with a small amount of silica-gel (Merck, Art 9385), was packed in a column, then subjected to the flash column chromatography with elution by chloroform-methanol (30 : 1), chloroform-methanol (20 : 1) and chloroform-methanol (15 : 1), in this order. A fraction eluted with chloroform-methanol (15 : 1) was dried in vacuo to obtain the product (234 mg; yield: 83 %).

The compound thus obtained has identical physicochemical properties to 6'-deoxy-6'-chloroneplanosin A in Example 1.

Example 3

Production of 6'-deoxy-6'-bromoneplanosin A:

Following the procedure of Example 2 carbon tetrachloride was replaced by carbon tetrabromide to obtain the product.

Yield: 216 mg (yield: 66 %)

MS (FAB) ; m/e 306, 328 ($MH^+$)

$^1$H-NMR (400 MHz, DMSO-d6) δ ; 4. 22 (d, 1H, H-6' b)、 4. 30 (d, 1H, H-6' a, $J_{6'ab'}$ = 11Hz)、 4. 37 (m、 1H, H-2')、 4. 57 (dd, 1H, H-3' , $J_{2',3'}$ = 5. 37 Hz)、 5. 19 (d, 1H, OH)、 5. 28 (d, 1H, OH)、 5. 37 (dd, 1H, H-1' , $J_{1',2'}$ = $J_{1',5'}$ = 2. 44 Hz)、 6. 04 (bs, 1H, H-5')、 7. 23 (bs, 2H, $NH_2$)、 8. 08 (bs, 1H, H-2)、 8. 15 (bs, 1H, H-8)

TLC ; Rf = 0. 32 [Merck, Art 5715 plate developing solvent: Chloroform-methanol (5 : 1)

Example 4

Production of 6'-deoxy-6'-fluoroneplanosin A:

2',3', -O-isopropylidene-neplanosin A (363 mg; 1.2 m mol) was suspended in dichloromethane (24 ml), the inside of the reaction vessel was replaced with argon gas, and diethylaminosulfur -trifluoride (375 μl; 2 equivalent) was poured therein and stirred at 0 °C for 1 hour. The reaction mixture was adjusted to room temperature, 0.75 N aqueous sodium bicarbonate solution (12 ml) was added, stirred for 3 minutes and chloroform (60 ml) was added thereto. Insoluble material was removed by filtration and the solution was washed with chloroform (30 ml). The filtrate was separated and the chloroform layer was filtered with Watman 1ps filter paper, then dried in vacuo. The residue, dissolved in a small amount of chloroform, was charged on a column (4 × 15 cm) of silica-gel (Wako Pure Chem. Co., C-200), then eluted with chloroform and chloroform-methanol (20 : 1). A fraction eluted with chloroform-methanol (20 : 1) was dried in vacuo to obtain 6'-deoxy-6'-fluoro-2',3'-O-isopropylidene neplanosin A, white powder, 123 mg. Yield: 34 %

MS (FAB) ; m/e 306 ($MH^+$)

$^1$H-NMR (400 MHz, $CDCl_3$ ) δ ; 1. 50、 1. 3 7 (each s, each 3H, isop- $CH_3$ × 2)、 4. 78 (d, 1H, H-2')、 5. 17 (ddd, 2H, H-6' , $J_{6',F}$ = 46. 9 Hz)、 5. 44 (d, 1H, H-3' , $J_{2',3'}$ = 5. 4 Hz)、 5. 61 (m, 1H, H-1')、 5. 62 (bs、 2H, $NH_2$) 5. 89 (m, 1H, H-5')、 7. 69 (s, 1H, H-2)、 8. 34 (s, 1H, H-8)

TLC ; Rf = 0. 72 [Merck, Art 5715 plate develo-ping solvent; Chloroform-methanol (5 : 1)

The above product (100 mg; 0.33 m mol) dissolved in 50 % formic acid (10 ml) was stirred at room temperature for 20 hours. The reaction solvent was removed in vacuo and water was added to the residue, then dried in vacuo. Tetrahydrofuran was added to the residue and the thus formed crystals were filtered to obtain the compound (31 mg). The filtered solution was further concentrated in vacuo. The residue was purified by preparative chromatography using a silica-gel plate (Merck, Art 5717) with a developer; chloroform-methanol (5 : 1) and elution; chloroform-methanol (2 : 1) to obtain the compound (38 mg).

Total yield: 69 mg (yield: 79.4%)

MS (FAB) ; m/e 266 ($MH^+$)

$^1$H-NMR (DMSO-d6 • $D_2O$, 400 MHz) δ ; 4. 38 (dd, 1H, H-2' , J = 5. 9, 5. 4 Hz)、 4. 51 (d, 1H, H-3' , J = 5. 4 Hz)、 5. 06 (dd, 1H, H-6' b, J = 13. 2, 46. 9 Hz)、 5. 13 (ddd, 1H, H-6a, J = 2. 0, 13. 2, 46. 9 Hz)、 5. 40 (m, 1H, H-1')、 5. 96 (dd, 1H, H-5' , J = 2. 9, 1. 5 Hz)、 8. 12, 8. 11 (each s, each 1H, H-2, H-8)

$^{13}$C-NMR (DMSO-d6, 100 MHz) δ ; 64. 18 ( C - 1')、 71. 48 (C-3')、 76. 11 (C-2')、 7 9. 83 (C-6' , $J_{6',F}$ = 161. 7 Hz)、 119. 11 (C - 5)、 127. 76 (C-5' , $J_{5',F}$ = 7. 6 Hz)、 139. 83 (C-8)、 143. 87 (C-4' , $J_{4',F}$ = 15. 2 Hz)、 149. 61 (C-4)、 152. 34 (C-2)、 155. 88 (C-6)

TLC ; Rf = 0. 24 [Merck, Art 5715 plate developing solvent; Chloroform-methanol (5 : 1)

Example 5

Production of 6'-deoxy-6'-iodoneplanosin A:

6'-deoxy-6'-chloroneplanosin A (28 mg; 0.1 m mol) and LiI (67 mg; 5 equivalent) were refluxed in acetonitrile (3 ml) for 15 minutes. The reaction mixture was ice-cooled and the solvent was removed in vacuo. The residue, dissolved in a small amount of methanol, was purified by preparative chromatography using a silica-gel plate (Merck, Art 5717) with developer; chloroform-methanol (5 : 1) and an elution; chloroform -methanol (2 : 1) to obtain the product (12 mg; yield: 32 %)

$^1H$-NMR ($CD_3$ OD, 90 MHz) $\delta$ ; 4.36 (dd 1H, H-2' , J = 4.7 Hz, 5.6 Hz)、 4.84 (d, 1H, H-3')、 5. 43 (m, 1H, H-1')、 6. 10 (m, 1H, H-5')、 7. 99 (s, 1H, H-2)、 8. 18 (s, 1H, H-8)
TLC ; Rf = 0. 38 [Merck, Art 5715 plate developing solvent; Chloroform-methanol (5 : 1)

## Claims

**1.** A compound of formula (1):

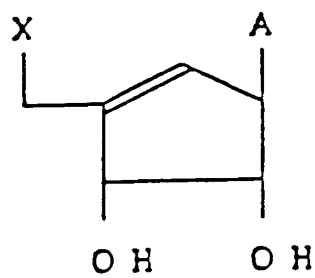

wherein A is an adenine-9-yl group and X is a halogen, or a pharmacologically acceptable non-toxic salt thereof.

**2.** A compound according to claim 1 which is in the form of a hydrochloride, sulfate, phosphate, acetate, propionate, tartrate, citrate, glycolate, gluconate, succinate, malate, glutomate, aspartate or methanesulfonate salt.

**3.** A process for the production of a compound as defined in claim 1 or 2 which comprises substituting the hydroxyl group at position 6′ in neplanosin A or 2′ and/or 3′-O-protected neplanosin A with a halogen by a halogenating agent, and, when the hydroxyl groups at positions 2′ and/or 3′ are protected, removing the protecting group thereof, and, if desired, converting the thus obtained compound of formula (1) to a pharmacologically acceptable non-toxic salt thereof.

**4.** A process according to claim 3 wherein the halogenating agent is triphenylphosphine and $CCl_4$ or $CBr_4$, $SOCl_2$, $SOBr_2$, diethylamino sulfatrifluoride or an alkaline metal iodide.

**5.** A process for the production of a compound as defined in claim 1 or 2 wherein X is iodine, which comprises substituting the halogen at position 6′ in a compound of formula:

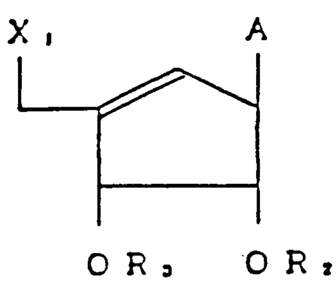

wherein A is adenine-9-yl, $X_1$ is chlorine or bromine and $R_2$ and $R_3$, which may be identical or different, are each hydrogen or a hydroxyl protecting group, with iodine by an iodinating agent, and, when the hydroxyl groups at positions 2′ and/or 3′ are protected, removing the protecting group thereof, and, if desired, converting the thus obtained compound of formula (1) in which X is iodine to a pharmacologically acceptable non-toxic salt thereof.

**6.** A compound as defined in claim 1 or 2 for use in a method of treatment of the human or animal body by therapy.

**7.** A compound as defined in claim 1 or 2 for use as an antitumor agent.

8. Use of a compound as defined in claim 1 or 2 in the manufacture of a medicament for use as an antitumor agent.

9. A pharmaceutical composition which comprises a compound as defined in claim 1 or 2 and a pharmaceutically acceptable diluent.

**Patentansprüche**

1. Verbindung der Formel (1):

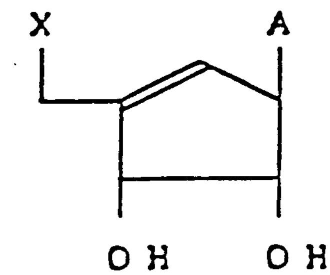

worin A eine Adenin-9-yl-Gruppe und X ein Halogenatom ist oder eines seiner pharmakologisch verträglichen nicht-toxischen Salze.

2. Verbindung nach Anspruch 1, dadurch gekennzeichnet, daß sie in Form eines Hydrochlorids, Sulfats, Phosphats, Acetats, Propionats, Tartrats, Zitrats, Glycolats, Gluconats, Sukzinats, Malats, Glutomats, Aspartats oder Methansulfonats als Salz vorliegt.

3. Verfahren zur Herstellung einer Verbindung nach Anspruch 1 oder 2, dadurch gekennzeichnet, daß man die Hydroxylgruppe in 6'-Stellung von Neplanosin A oder von 2'- und/oder 3'-O-geschütztem Neplanosin A mit einem Halogenatom mit Hilfe eines Halogenierungsmittels substituiert und (sofern die Hydroxylgruppen in 2'-Stellung und/oder 3'-Stellung geschützt sind) deren Schutzgruppen entfernt und gegebenenfalls die auf diese Weise erhaltene Verbindung der Formel (1) in ein pharmakologisch verträgliches nicht-toxisches Salz überführt.

4. Verfahren nach Anspruch 3, dadurch gekennzeichnet, daß das Halogenierungsmittel Triphenylphosphin und $CCl_4$ oder $CBr_4$, $SOCl_2$, $SOBr_2$, Diethylaminosulfatrifluorid oder ein Alkalimetalliodid ist.

5. Verfahren zur Herstellung einer Verbindung nach Anspruch 1 oder 2, worin X ein Iodatom ist, dadurch gekennzeichnet, daß man das Halogenatom in 6'-Stellung einer Verbindung der folgenden Formel

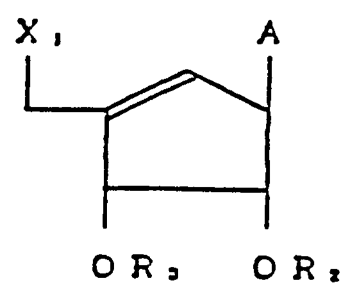

worin A eine Adenin-9-yl-Gruppe, $X_1$ ein Chloratom oder Bromatom und $R_2$ und $R_3$, die gleich oder verschieden sein können, jeweils ein Wasserstoffatom oder eine Hydroxyl-Schutzgruppe bedeuten, durch ein Iodatom mit einem Iodierungsmittel substituiert und (sofern die Hydroxylgruppen in 2'-Stellung und/oder 3'-Stellung geschützt sind) deren Schutzgruppen entfernt und gegebenenfalls die auf diese Weise erhaltene Verbindung der Formel (1), in der X ein Iodatom bedeutet, in ein pharmakologisch verträgliches nicht-toxisches Salz überführt.

6. Verbindung nach Anspruch 1 oder 2 zur Verwendung in einem Verfahren zur therapeutischen Behandlung des menschlichen oder tierischen Körpers.

7. Verbindung nach Anspruch 1 oder 2 zur Verwendung als Antitumormittel.

8. Verwendung einer Verbindung nach Anspruch 1 oder 2 zur Herstellung eines Medikaments zur Verwendung als Antitumormittel.

9. Pharmazeutische Zusammensetzung, gekennzeichnet durch eine Verbindung nach Anspruch 1 oder 2 und ein pharmazeutisch verträgliches Verdünnungsmittel.

**Revendications**

1. Composé de la formule (1) :

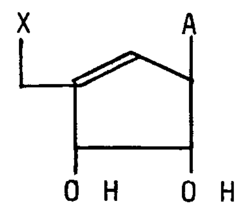

où A est un groupe adénine-9-yle et X est un halogène, ou son sel non toxique pharmacologiquement acceptable.

2. Composé selon la revendication 1 qui est sous la forme d'un sel de chlorhydrate, sulfate, phosphate, acétate, propionate, tartrate, citrate, glycolate, gluconate, succinate, malate, glutomate, aspartate ou méthane sulfonate.

3. Procédé pour la production d'un composé tel que défini dans la revendication 1 ou 2 qui comprend la substitution du groupe hydroxyle à la position 6' dans la néplanosine A ou la néplanosine A 2' et/ou 3'-0 protégée par un halogène au moyen d'un agent halogénant et, quand les groupes hydroxyles aux positions 2' et/ou 3' sont protégés, l'élimination du groupe protecteur et, si on le souhaite, la conversion du composé ainsi obtenu de formule (1) en un sel non toxique pharmacologiquement acceptable.

4. Procédé selon la revendication 3, où l'agent halogénant est la triphénylphosphine et $CCl_4$ ou $CBr_4$, $SOCl_2$, $SOBr_2$, le diéthylamino sulfatrifluorure ou un iodure d'un métal alcalin.

5. Procédé pour la production d'un composé tel que défini à la revendication 1 ou 2, où X est iode, qui comprend la substitution de l'halogène à la position 6' dans un composé de formule :

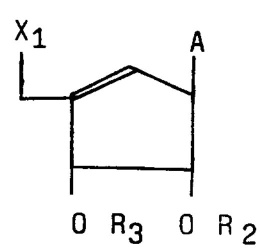

où A est adénine-9-yle, $X_1$ est chlore ou brome et $R_2$ et $R_3$, qui peuvent être identiques ou différents, sont chacun hydrogène ou un groupe hydroxyle protecteur par de l'iode par un agent iodant et, lorsque les groupes hydroxyles aux positions 2' et/ou 3' sont protégés, l'élimination du groupe protecteur et, si on le souhaite, la conversion du composé ainsi obtenu de formule (1) où X est iode en un sel non toxique pharmacologiquement acceptable.

**6.** Composé tel que défini à la revendication 1 ou 2, pour une utilisation dans une méthode de traitement du corps humain ou animal par thérapie.

**7.** Composé tel que défini à la revendication 1 ou 2, pour une utilisation comme agent anti-tumeur.

**8.** Utilisation d'un composé tel que défini dans la revendication 1 ou 2 dans la fabrication d'un médicament à utiliser comme agent anti-tumeur.

**9.** Composition pharmaceutique qui comprend un composé tel que défini à la revendication 1 ou 2 et son diluant acceptable en pharmacie.